(19) Europäisches Patentamt / European Patent Office / Office européen des brevets

(11) **EP 3 203 930 B1**

(12) **EUROPEAN PATENT SPECIFICATION**

(45) Date of publication and mention
of the grant of the patent:
**13.05.2020 Bulletin 2020/20**

(21) Application number: **15849620.8**

(22) Date of filing: **06.10.2015**

(51) Int Cl.:
**A61F 2/02** (2006.01)

(86) International application number:
**PCT/US2015/054149**

(87) International publication number:
**WO 2016/057462 (14.04.2016 Gazette 2016/15)**

(54) **TISSUE INTEGRATION DEVICES AND METHODS OF MAKING THE SAME**

GEWEBEINTEGRATIONSVORRICHTUNGEN UND VERFAHREN ZUR HERSTELLUNG DAVON

DISPOSITIFS D'INTÉGRATION TISSULAIRE ET MÉTHODES DE FABRICATION ASSOCIÉES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.10.2014 US 201462060181 P**

(43) Date of publication of application:
**16.08.2017 Bulletin 2017/33**

(73) Proprietor: **Proxy Biomedical Inc.**
**Cleveland, Ohio 44103 (US)**

(72) Inventors:
• **DURKIN, Tony**
**Knocknacarra (IE)**
• **KING, Dean**
**Knocknacarra (IE)**
• **NUGENT, Barry**
**Galway H91 C2NF (IE)**

• **MULROONEY, Peter**
**Galway H91 C2NF (IE)**
• **MOLONEY, Kieran**
**Galway (IE)**
• **GINGRAS, Peter H.**
**Cleveland, Ohio 44103 (US)**

(74) Representative: **FRKelly**
**27 Clyde Road**
**Dublin D04 F838 (IE)**

(56) References cited:
**WO-A1-2013/030446       WO-A1-2015/052346**
**US-A1- 2005 170 012      US-A1- 2006 141 012**
**US-A1- 2006 141 012      US-A1- 2008 081 323**
**US-A1- 2008 234 730      US-A1- 2009 216 338**
**US-A1- 2009 216 338      US-A1- 2010 042 215**
**US-A1- 2010 121 446      US-B1- 6 358 284**

**Description**

**Field of the Invention**

A tissue integration device includes a biocomposite mixture of a polymer and a homogenously distributed tissue growth-promoting medium for the purpose of facilitating and speeding controlled tissue formation and integration across and within the tissue integration device. Specifically disclosed are methods for manufacturing such devices, for example, but in no way limited to, tissue integration devices or the like.

**Background**

Many surgical procedures require the use of internal tethering of damaged bone or other tissue in order to fix the bone or tissue in position to address a misalignment, instability, and/or to promote the healing process, for example allowing bone or tendon regrowth at a damaged area. To facilitate such tethering it is often necessary to employ some form of bone or tissue anchor or fixation device to serve as a fixed point to which a suture may be anchored, to allow a tendon or ligament to be sutured and secured to the bone. For instance, when performing a rotator cuff repair or the like, the device may be threaded into a suitable hole formed in a section of bone and secured by a suture to achieve fixation of tendons or ligaments to the respective bone. Fixation or tissue repair devices may also be used to enable additional surgical apparatus to be secured as required.

Such fixation or tissue repair devices may take many forms. For example, an externally threaded bone screw or buttons or plugs are used for soft tissue repair such as upper and lower extremities, pins or darts for meniscal repair or small bone fracture fixation, plates for cranio maxilla facial fracture fixation, nails for fracture fixation, scaffolds for dental or bone grafts, tacks for implant, scaffolds for osteonecrosis, etc.

Commercially produced surgical fixation tools such as screws, pins, anchors, etc. are typically injection molded from a suitable material such as a polymer or the like. These injection molded parts can be manufactured from a single resin or a polymer mixture, which is typically in the form of resin combined with a growth promoting medium, for example tricalcium phosphate (TCP) or any other suitable alternative. In many instances and prior-art devices the polymer employed is biodegradable and when combined with a bioactive material effectively promotes bone or tissue ingrowth thus accelerating the healing process. Despite the development of three-dimensional scaffolds that combine biodegradability and bioactive substances to promote tissue and bone growth at the site of injury, the single biggest challenge has been to improve the mechanical strength of these devices. Better mechanical strength can result in devices with much less material in the human body and further allows addition of more features in these devices, such as optimized structures with pores, vented holes, etc. US2006141012 A1 discloses tissue scaffolds and methods of making and using these scaffolds for tissue engineering, the scaffolds comprising interconnecting pores that are created by attaching a plurality of pore containing films onto each other such that pores of a first film interconnect with the pores of a second film to define pathways extending from the first film to the second film. Figure 5C of this document shows such interconnecting pores, the pores however not being line of sight pores in view of two hydrogel film layers (126) obstructing the pores.

**Summary**

The invention is defined in the appended claims. One aspect of the present disclosure relates to a tissue integration device. The tissue integration device can be produced by forming a polymer mixture into a shape. The polymer mixture can comprise a polymer resin and a growth-promoting medium. Next, at least one polymer comprising the polymer resin can be oriented in at least one direction. The shaped polymeric material can then be formed into the tissue integration device.

Another aspect of the present disclosure relates to a tissue integration device. The tissue integration device can be produced by forming a polymer mixture into a shape. The polymer mixture can comprise a polymer resin and a growth-promoting medium. Next, at least one polymer comprising the polymer resin can be oriented in at least one direction. When the polymer is oriented, the polymer chains become more aligned in the direction of orientation and improve the mechanical properties of the shaped polymeric mixture or material. The shaped polymeric material can then be formed into the tissue integration device. One or more line-of-sight pores can be formed in the tissue integration device. The one or more line-of-sight pores are sized and dimensioned to promote tissue ingrowth when the tissue integration device is implanted in a subject.

Another aspect of the present disclosure relates to a method for forming a tissue integration device. The method can comprise the steps of: forming a polymer mixture that includes a polymer resin and a growth-promoting medium; processing the polymer mixture into a shape selected from the group consisting of a plaque, a sheet, a film and a strip; orienting at least one polymer comprising the polymer resin in at least one direction; annealing the shaped polymer mixture; cutting the annealed, shaped polymer mixture into at least one strip; wrapping the at least one strip around a mandrel to form a tube of concentric annular layers; compressing the layers together to laminate the layers; and optionally forming one or more pores in the tissue integration device, wherein the one or more pores are sized and dimensioned to promote tissue ingrowth when the tissue integration device is implanted in a subject.

## Description of the Drawings

The accompanying drawings, which are incorporated in and constitute a part of the specification, illustrate various example systems, methods, and so on, that illustrate various example embodiments of aspects of the present disclosure. It will be appreciated that the illustrated element boundaries (e.g., boxes, groups of boxes, or other shapes) in the figures represent one example of the boundaries. One of ordinary skill in the art will appreciate that one element may be designed as multiple elements or that multiple elements may be designed as one element. An element shown as an internal component of another element may be implemented as an external component and vice-versa. Furthermore, elements may not be drawn to scale.

The present disclosure will now be described with reference to the accompanying drawings, in which:

Fig. 1 is a process flow diagram illustrating a method for forming a tissue integration device according to one aspect of the present disclosure;

Fig. 2 is a photograph of a polymer mixture in a mold before an initial film comprising the polymer mixture is cast;

Fig. 3 is a photograph of the polymer mixture in Fig. 2, formed into films, as they appear after they have been cast;

Fig. 4 is a perspective view showing a film in Fig. 3 being subjected to biaxial stretching;

Fig. 5A is a perspective view of the film in Fig. 4 partially wound onto a mandrel;

Fig. 5B is a perspective view of the film in Fig. 5A fully wound onto the mandrel;

Fig. 6 is a sectioned view of the mandrel in Fig. 5B contained within a compression tool;

Fig. 7 is a perspective view of the compression tool in Fig. 6 in an open configuration;

Fig. 8 is a perspective view showing one example of a tissue integration device which can be formed according to the disclosed methods;

Fig. 9A is a side view showing another example of a tissue integration device which can be formed according to the disclosed methods;

Fig. 9B is a perspective view of the tissue integration device in Fig. 9A;

Fig. 10A is a perspective view showing another example of a tissue integration device which can be formed according to the disclosed methods;

Fig. 10B is a cross-sectional view taken along Line 10B-10B in Fig. 10A;

Fig. 11 is an image showing one example of a tissue integration formed according to the present disclosure (left) and a market leading predicate product (right); and

Fig. 12 is a graph showing comparing torsional strength of tissue integration devices formed according to the present disclosure and a market leading predicate product.

## Detailed Description

Unless defined otherwise, all technical and scientific terms used herein have the same meaning as is commonly understood by one of skill in the art to which the present disclosure pertains.

In the context of the present disclosure, the singular forms "a," "an" and "the" can include the plural forms as well, unless the context clearly indicates otherwise. It will be further understood that the terms "comprises" and/or "comprising," as used herein, can specify the presence of stated features, steps, operations, elements, and/or components, but do not preclude the presence or addition of one or more other features, steps, operations, elements, components, and/or groups thereof.

As used herein, the term "and/or" can include any and all combinations of one or more of the associated listed items.

As used herein, phrases such as "between X and Y" and "between about X and Y" can be interpreted to include X and Y.

As used herein, phrases such as "between about X and Y" can mean "between about X and about Y."

As used herein, phrases such as "from about X to Y" can mean "from about X to about Y."

It will be understood that when an element is referred to as being "on," "attached" to, "connected" to, "coupled" with, "contacting," etc., another element, it can be directly on, attached to, connected to, coupled with or contacting the other element or intervening elements may also be present. In contrast, when an element is referred to as being, for example, "directly on," "directly attached" to, "directly connected" to, "directly coupled" with or "directly contacting" another element, there are no intervening elements present. It will also be appreciated by those of skill in the art that references to a structure or feature that is disposed "adjacent" another feature may have portions that overlap or underlie the adjacent feature.

Spatially relative terms, such as "under," "below," "lower," "over," "upper" and the like, may be used herein for ease of description to describe one element or feature's relationship to another element(s) or feature(s) as illustrated in the figures. It will be understood that the spatially relative terms can encompass different orientations of the apparatus in use or operation in addition to the orientation depicted in the figures. For example, if the apparatus in the figures is inverted, elements described as "under" or "beneath" other elements or features would then be oriented "over" the other elements or features.

It will be understood that, although the terms "first," "second," etc. may be used herein to describe various elements, these elements should not be limited by these terms. These terms are only used to distinguish one element from another. Thus, a "first" element discussed below could also be termed a "second" element without departing from the teachings of the present disclosure. The sequence of operations (or steps) is not limited to the order presented in the claims or figures unless specifically indicated otherwise.

As used herein, the terms "subject" and "patient" can be used interchangeably and refer to any warm-blooded organism including, but not limited to, human beings, pigs, rats, mice, dogs, goats, sheep, horses, monkeys, apes, rabbits, cattle, etc.

As used herein, the term "tissue" in the context of a tissue integration device can refer to any biological tissue, such as bone, cartilage, tendon, connective tissue, or muscle that a tissue integration device can be fixed onto and/or into.

As used herein, the term "tissue integration device" can include any device or combination of devices formed according to the methods of the present disclosure. Such devices can have a variety of forms including, but not limited to, a suture anchor, a surgical screw, a tack, a pin, a rod or the like, in addition to a bone or tissue scaffold.

As used herein, the term "polymer resin" can refer to a macroscopic mass of material comprising a plurality of polymer molecules, as opposed to the individual microscopic polymer molecules.

As used herein, the term "line-of-sight", when referring to pores formed in the tissue integration devices of the present disclosure, refers to pores that form an unobstructed and visible path between inner and outer surfaces of a tissue integration device according to the present disclosure.

As used herein, the terms "homogenously distributed" or "homogenous distribution" can refer to a material (*e.g.,* a polymer mixture) or device (*e.g.,* a tissue integration device) that has uniform physical properties (*e.g.,* mechanical strength) and/or chemical properties with substantially the same composition throughout.

As used herein, the term "stem cell" can refer to a cell that can undergo self-renewal (*i.e.,* progeny with the same differentiation potential) and also produce progeny cells that are more restricted in differentiation potential. Within the context of the invention, a stem cell would also encompass a more differentiated cell that has de-differentiated, for example, by nuclear transfer, by fusion with a more primitive stem cell, by introduction of specific transcription factors, or by culture under specific conditions. One example of a stem cell is a mesenchymal stem cell (MSC). Other examples of stem cells that may be used as part of the devices and methods disclosed herein are described in U.S. Patent Application Publication No. 2011/0020292 A1 to Van't Hof.

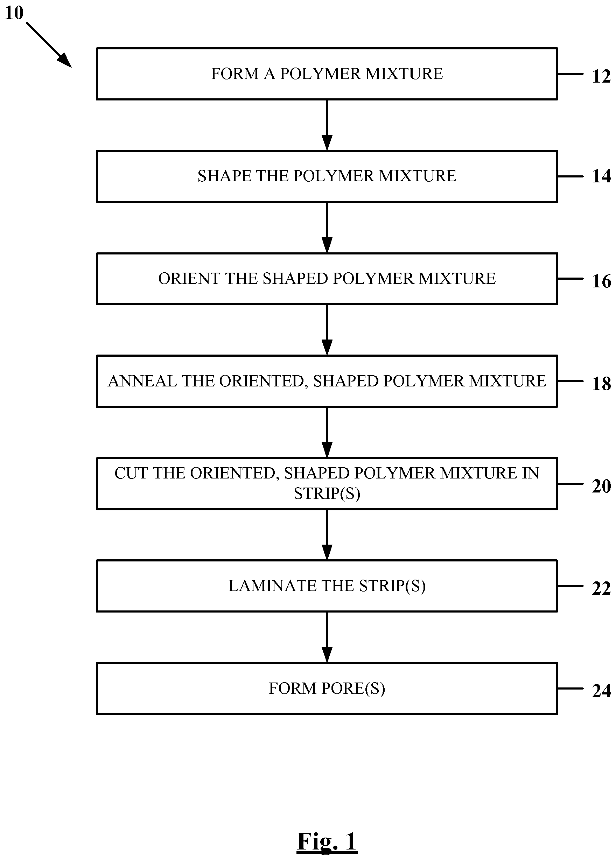

One aspect of the present disclosure includes a method 10 (Fig. 1) for forming a tissue integration device. The method 10 is illustrated as process flow diagram with flowchart illustrations. For purposes of simplicity, the method 10 is shown and described as being executed serially or sequentially; however, it is to be understood and appreciated that the present disclosure is not limited by the illustrated order as some steps could occur in different orders and/or concurrently with other steps shown and described herein. Moreover, not all illustrated aspects may be required to implement the method 10.

At Step 12 of the method 10, a polymer mixture is formed. In some instances, the polymer mixture can be referred to herein as a "biocomposite" or a "polymer blend". The polymer mixture can be formed by mixing a polymer resin and a tissue growth-promoting medium or bio-ceramic. The polymer resin can include at least one natural or synthetic polymer that is bio-absorbable or non-absorbable. Non-limiting examples of polymers can include polylactides (*e.g.,* poly(lactic acid) (PLA), poly L lactic acid (PLLA), or PLDLA 7030), ultra-high-molecular-weight polyethylene (UHMWPE), polyether ether ketone (PEEK), polyurethanes (PU), polytetrafluoroethylene (PTFE), polydioxanone (PDO), poly(epsilon-caprolactone) (PCL), polyorthoester, poly(2-hydroxy-ethyl-methacrylate (PHEMA), polyhydroxy butyrate (PHB), and combinations or mixtures thereof.

The tissue growth-promoting medium or bio-ceramic can include any agent, moiety, or compound capable of promoting tissue ingrowth. Non-limiting examples of tissue growth-promoting media or bio-ceramics can include, but are not limited to, hydroxyapatite (HA), bioactive glasses (BGs), $\alpha$-tricalcium phosphate ($\alpha$TCP), $\beta$-tricalcium phosphate (($\beta$TCP), their derivatives and combinations thereof.

The ratio of the polymer resin to the tissue growth-promoting medium or bio-ceramic in the polymer mixture (in w/w) can range from about 70%:30%, or about 75%:25%, or about 80%:20%, or about 85%:15%, or about 90%:10%, or about 95%:5%, or about 99%:1 %. In one example, the ratio of the polymer resin to the tissue growth-promoting medium or bio-ceramic in the polymer mixture can be 85% w/w PLA and 15% w/w $\beta$TCP. In another example, the ratio of the polymer resin to the tissue growth-promoting medium or bio-ceramic in the polymer mixture can be 85% w/w PLLA and 15% w/w $\beta$TCP. It will be appreciated by those skilled in the art that the polymer resin can be mixed with the tissue growth-promoting medium or bio-ceramic at different ratios as desired to produce a tissue integration device of superior mechanical strength.

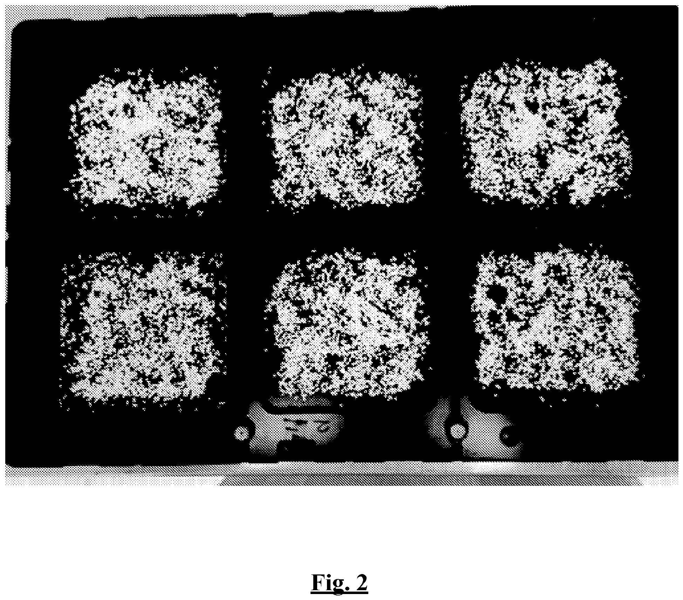

After forming the polymer mixture, the polymer mixture is placed in a mold (Fig. 2). The mold can have any desired shape (*e.g.,* rectangular, square, circular, etc.) and dimensions.

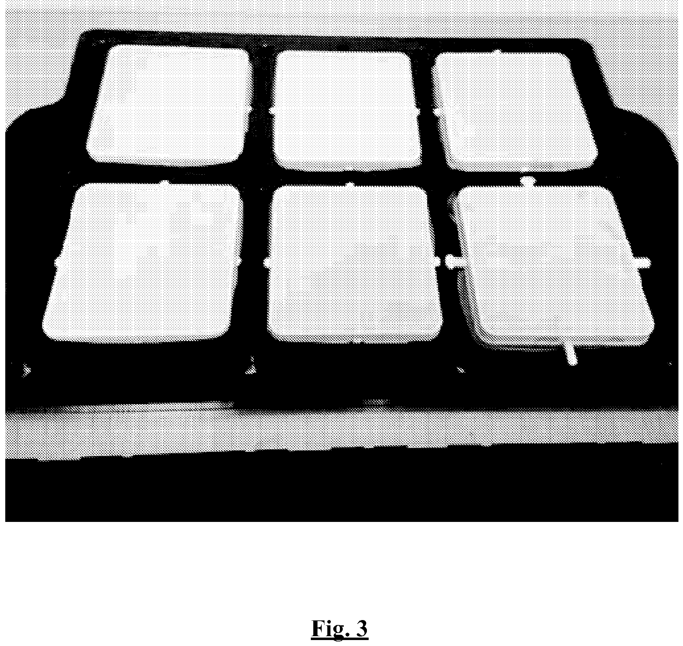

Next, at Step 14, the polymer mixture is removed from the mold and formed into a shaped polymer mixture using one or combination of known processing techniques, such as casting, injection molding, extrusion, transfer molding, thermoforming, rotational molding, blow molding, and the like (Fig. 3). Examples of shapes into which the polymer mixture can be formed include sheets, films (*e.g.,* thin films), strips, and plaques. In one example, the polymer mixture can be formed into a plaque, film, or sheet by compression molding. It will be appreciated by one of ordinary skill in the art that the film or sheet thickness can be adjusted as desired based on the dimensions of the mold cavity. Advantageously, blending or mixing the polymer resin with the tissue growth-promoting medium or bioceramic (*e.g.,* $\beta$TCP) before processing (*e.g.,* casting) ensures that the tissue growth-promoting medium or bioceramic (*e.g.,* $\beta$TCP) is homogenously distributed in the resulting plaque or sheet. The homogenous distribution of the tissue growth-promoting medium or bioceramic promotes uniform tissue integration into the tissue integration device (when implanted in a subject), as well as imparting the tissue integration device with improved mechanical properties. The homogenous distribution of tissue growth-promoting medium or bioceramic in the shaped polymeric mixture can be determined visually by using any acceptable microscopy or scanning procedure known to those of skill in the art, such as SEM or CT scanning.

At Step 16, at least one polymer comprising the polymer resin in the shaped polymer mixture is subjected to a orienting or a stretching step. Orienting the polymer, including polymer chains that make up the polymer resin, in the shaped polymer mixture improves the mechanical properties (*e.g.,* torsional strength, tensile strength and elongation) and structural properties (*e.g.,* orientation and crystallinity) thereof. The terms "orienting" and "stretching" can be used interchangeably herein and refer to establishing the optimal or maximum or desired or highest stretch ratio target for a polymer. The highest stretch ratio refers to a ratio where the polymer is stretched to its breaking point, whereas the optimal or maximum or desired stretch ratio is a ratio at which the polymer comprising the polymer resin is stretched to almost its breaking point. The stretch ratio target for a given polymer is derived by taking each polymer/material in isolation and challenging the capabilities of that polymer/material until almost its breaking point. Specifically, the polymer is converted into a thin film and then stretched under defined conditions of temperature, speed (stretch rate) and draw-forces. The maximum stretch ratio of a shaped polymer mixture is also dependent on (i) the quality of the film, *i.e.,* free from nicks/cuts, micro cracks, air entrapments/holes, uniform film thickness, and quality film homogeneity before stretching, and (ii) target thickness of the shaped polymer mixture after stretching. For example, if the desired target thickness of a shaped polymer mixture after stretching is 0.05 mm, and its thickness before stretching is 1 mm, then the stretch ratio is calculated as follows:

$$\sqrt{(\text{Pre-Stretched Film thickness/Finished Target Bi-Axially Stretched film thickness})} *$$
$$\sqrt{(1/0.05)} = 4.47 \text{x} 4.47 \ (19.98)$$

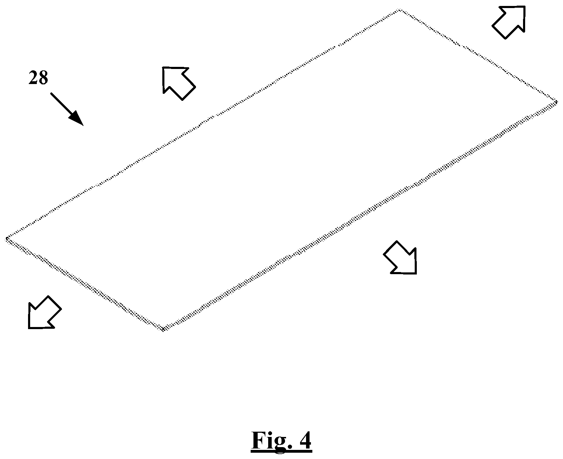

When the shaped polymer mixture is stretched in only one direction, *i.e.,* along its x-axis, then the shaped polymer mixture is said to be stretched uniaxially. When the shaped polymer mixture is stretched along both the x-axis and the y-axis, then the shaped polymeric mixture is said to be stretched biaxially (Fig. 4). The shaped polymer mixture may be stretched in one or both directions independently, or in both directions simultaneously. Once the optimal or maximum desired stretch ratio for a shaped polymer mixture is determined, the same process is repeated for a new shaped polymer mixture containing the same components, but the new shaped polymer mixture is stretched not until its breaking point, but until the stretch ratio of the determined target is reached. This stretch ratio is the maximum orientation point (or optimal stretch ratio) that can be achieved (without breaking), which is then mechanically categorized as the Ultimate Break Force and the Elongation percentage.

It will be appreciated by one of ordinary skill in the art that the stretch ratio can be varied in the x and y directions to optimize mechanical properties in a given direction. In one example, a shaped polymer mixture can be stretched at least four times more along the x-axis than it is stretched along the y-axis (or vice-versa). In another example, a shaped polymer mixture can be stretched biaxially at a ratio that is not 1:1.

By stretching the shaped polymeric mixture, either uniaxially or biaxially, the polymer chains forming the shaped polymeric mixture are both orientated to align with the axis along which the shaped polymeric mixture is stretched, in addition to being compressed into closer proximity with one another so as to give a higher density of polymer chains. Thus, while the stretched polymeric mixture will have a reduced thickness, it will have a higher density of polymer chains, which are also "oriented". Where the shaped polymer mixture is biaxially stretched, the chains will tend to orient themselves radially. It was surprisingly found that stretching a shaped polymer mixture biaxially provides a more uniform distribution of the tissue growth-promoting medium or bio-ceramic (*e.g.,* βTCP) throughout the polymer mixture, in addition to imparting superior strength and load (or mechanical) characteristics to the final tissue integration device when compared to a non-oriented or non-stretched polymer mixture.

At Step 18, the stretched polymer mixture is allowed to anneal. The annealing process allows the stretched polymer mixture (*e.g.,* the polymers) to relax to a specific target percentage. In one example, the stretched polymer mixture is subjected to a temperature of about 80°C. to about 250°C. for a period of about 30 seconds to about 5 minutes (*e.g.,* about 2 minutes). The annealing process imparts the stretched polymer mixture with superior performance characteristics in later stages of processing.

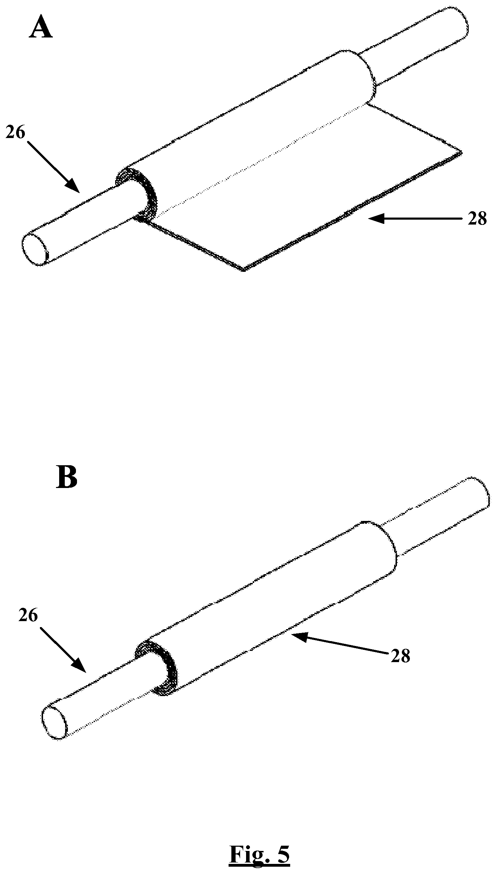

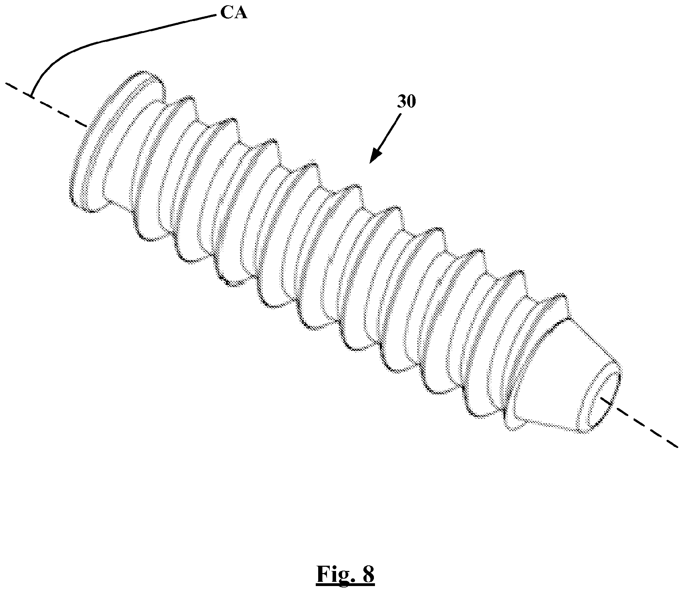

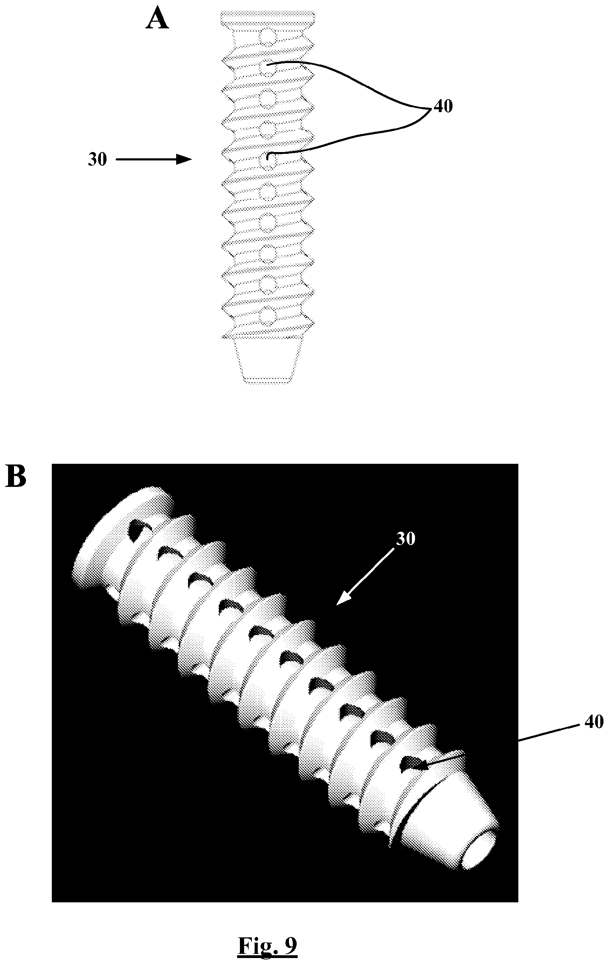

At Step 20, the annealed and shaped polymer mixture is cut into one or more strips. Fig. 4 illustrates an annealed, shaped polymer mixture that is cut into a single strip. In some instances, a strip is wrapped around a mandrel to form a tube of concentric annular layers. While multiple layers of the strip may be layered in numerous different configurations utilizing numerous different methodologies, one example of the method 10 is described with reference to Figs. 5A-7. Referring to Fig. 5A, a mandrel 26 is illustrated onto which a single strip 28 is partially wound. Multiple strips 28 can be wound onto the mandrel 26; however, it is preferable to simply size the strip such that only a single strip is required to provide the necessary shape and volume of the ultimate tissue integration device 30 (Figs. 8-10B). In this way, it will be appreciated that multiple essentially concentric layers are formed around the mandrel 26. Fig. 5B illustrates the mandrel 26 when the strip 28 has been fully wrapped thereabout to form a tubular construct of concentric annular layers.

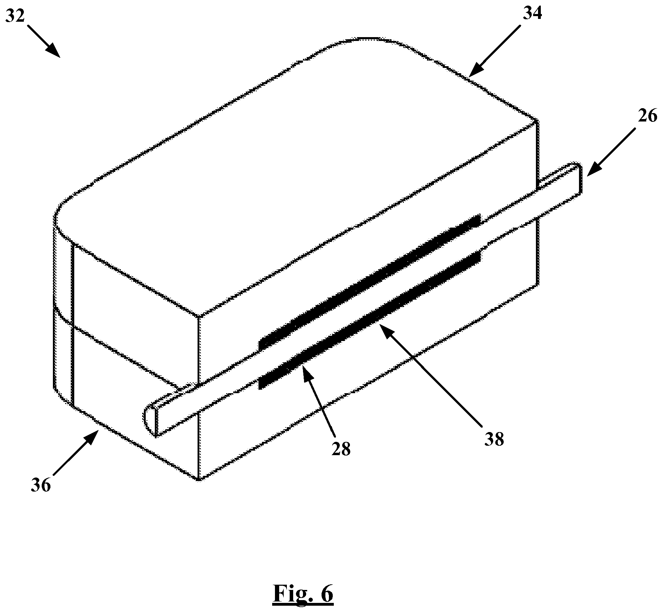

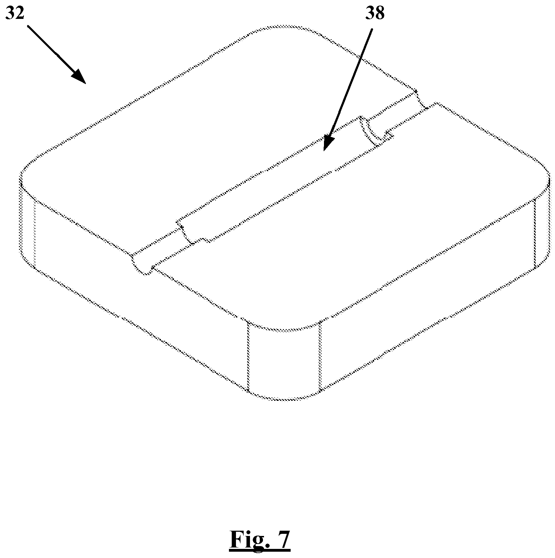

At Step 22, the fully wound mandrel 26 is placed into a suitable compression tool 32 (Fig. 6) having a first half 34 and a second half 36. The compression tool 32 is made from a block of material (*e.g.,* metal) having a groove or cavity 38 in its middle that is shaped and dimensioned to receive the fully wound mandrel 26 (Fig. 7). Thus, the halves 34, 36 are suitably separated and the fully wound mandrel 26 located therebetween and aligned or seated into one side of the cavity 38. The compression tool cavity 38 may contain specific geometry that is imparted onto the laminated structure. This geometry may create an outer surface or surfaces defining particular geometric shapes or contours, such as full or partial screw threads, barb grip features, tapers, surface textures, or any other form appropriate for use with the disclosed applications.

The halves 34, 36 are then closed, whereafter pressure and heat are applied for a specified time to completely or fully laminate the wrapped layers of the strip 28. For example, one or more cycles of heating followed by pressure and cooling phases can be applied to the to the fully wound mandrel 26. In some instances, depending on the polymer resin comprising the polymer mixture, each cycle can be about 5-60 minutes (*e.g.,* 20 minutes) in duration and include a temperature that ranges from about 5-300°C. (*e.g.,* 180°C. to 200°C.) and a pressure of 5-282 N/cm$^2$ (*e.g.,* 60 N/cm$^2$). The fully wound mandrel 26 can then be cooled for at least about 15 minutes following compression to permit re-alignment of the polymer chains. This step (Step F) of the method 10 produces a fully cross-laminated structure (*e.g.,* a multi-layered tissue integration device 30). It will be appreciated that any other functional alternative to the compression tool 32 may be employed to achieve the above-described lamination. After a desired period of time, the compression tool 32 is disassembled and the resulting structure (*i.e.,* a tissue integration device 30) removed from the cavity 38. The tissue integration device 30 is ready for surgical implantation or, as discussed below, may be further processed.

At Step 24, the method 10 can additionally or optionally include forming one or more pores 40 in the tissue integration device 30. The one or more pores 40 are sized and dimensioned to promote tissue ingrowth when the tissue integration device 30 is implanted in a subject. Step 24 involves strategic removal of material from the tissue integration device 30 by, for example, laser cutting, rotary die, perforation equipment, and/or any other suitable alternative. It will be appreciated that the shaped polymeric mixture could be initially produced with the porosity integrally formed therein, as described in PCT Publication No. WO 2015/052346 A1 to Durkin et al.*,* thus avoiding the additional step of removing material to produce the porosity. Individual pores 40 formed in the tissue integration device 30 may vary in size and/or shape to achieve a desired functionality in the device. For example, the pores 40 may be structurally discrete or distinct from one another or, alternatively, intersect one another at one or more points. In addition, the particular distribution of the pores 40 may be varied as required.

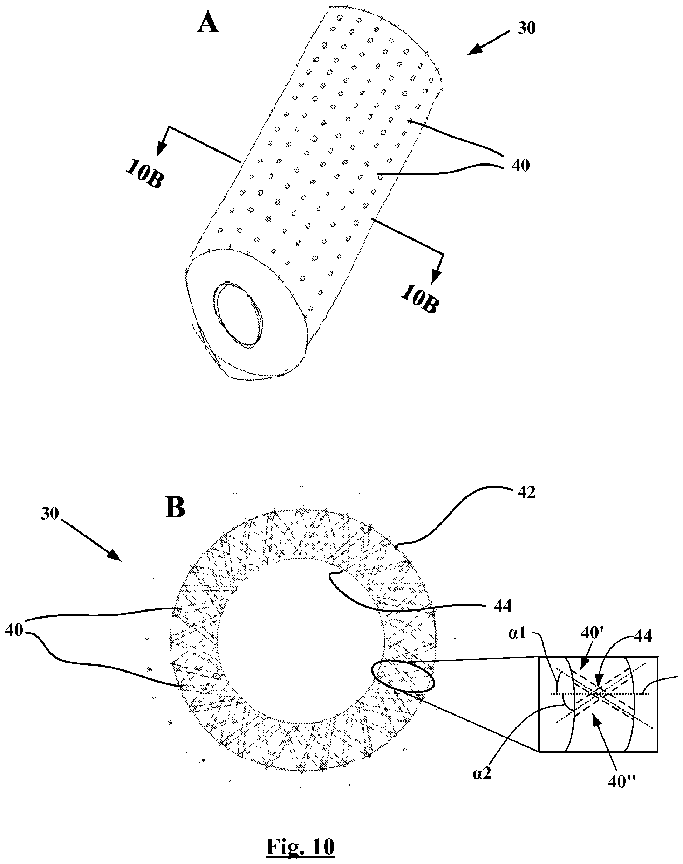

In some instances, the pores 40 can extend from an exterior surface 42 (Figs. 10A-B) of the tissue integration device 30 to an interior surface 44 thereof. In such instances, the pores 40 may serve as passageways to facilitate tissue ingrowth and establish a robust interface between the tissue integration device 30 and the surrounding bone or tissue once the device has been implanted. In other instances, the pores 40 extend only partially from the exterior surface 42 to the interior surface 44, thereby creating a "dead-end" or blind pore structure. In this instance, the "dead-end" pore structure can facilitate retention of biological agents (*e.g.,* stem cells, growth factors, etc.) within the pores 40 during and after implantation of the tissue integration device 30. In further instances, the pores 40 can be created at specific angles (*e.g.,* relative to a central axis CA of the tissue integration device 30) to provide a level of controlled inter-connectivity between the pores. For example, one or more line-of-sight pores 40 can be formed in a tissue integration device 30.

The line-of-sight pores 40 can be formed in the tissue integration device 30 to permit organized intersections between two or more of the pores, thereby providing a high degree of controlled connectivity amongst the pores. The high degree of connectivity facilitates tissue ingrowth as it provides a large surface area to promote cell adhesion and growth. As shown in the exploded region of Fig. 10B, one example of a tissue integration device 30 can include a series of line-of-sight pores 40 extending through the device in alternating directions, which provides one or more intersections between each of the pores. A first line-of-sight pore 40' can extend at an angle $\alpha1$ relative to an axis A, while a second line-of-sight pore 40" can extend at a second angle $\alpha2$ relative to the axis A. The angles $\alpha1$ and $\alpha2$ can be the same or different. In one example, each angle $\alpha1$ and $\alpha2$ is an acute angle, *e.g.,* about 45° or less. In some instances, a more acute angle (*e.g.,* 45° or less) will be preferable as it provides more surface area than a less acute angle (*e.g.,* 45° or more). The first and second line-of-sight pores 40' and 40" intersect at a common point 46.

Because the method 10 disclosed herein facilitates increased mechanical strength in the produced tissue integration devices, more material can be removed to form interconnected pores 40, larger vented holes, etc., while still meeting the strength requirements of the device 30. This is unlike conventional tissue integration devices, which are injection molded and thus lose significant mechanical strength upon removal of material therefrom. The ability of the present method 10 to provide controlled porosity can not only speed healing and improve the quality of tissue deposited at the implant site, but also permit tissue formation (*e.g.,* vasculature) with sufficient vascularity to sustain viable tissue (*e.g.,* in a patient suffering from osteonecrosis). Advantageously, tissue integration devices 30 formed by the present method 10 can have large vented holes, for example, which permit bone marrow and blood to readily flow through the device and promote tissue ingrowth while still maintaining the mechanical strength required for high stress clinical applications.

Exemplary embodiments of tissue integration devices 30 formed according to the method 10 are illustrated in Figs. 8-9B. Such tissue integration devices 30 can be used in surgical procedures, for example, to secure or anchor tissue, bone or the like, as well as secure additional surgical apparatus to bone, cartilage or other suitable tissue, while promoting tissue in-growth in the devices.

The following examples are for the purpose of illustration only and are not intended to limit the scope of the claims, which are appended hereto.

## EXAMPLES

### Example 1. General method for making a tissue integration device having biaxially oriented polymers

Commercially available, medical grade polymer material that is bio-absorbable is mixed or blended with a tissue growth-promoting medium (*e.g.,* $\beta$TCP) at a ratio of 85 % w/w polymer to 15% w/w tissue growth-promoting medium and compounded into a pellet form. The blended polymer mixture is then cast into a film or fed into an extruder through a hopper and conveyed forward by a feeding screw, where it is forced through a die to convert the pellets into a continuous polymer mixture. Heating elements applied to the polymer mixture soften and melt the mixture. The resulting polymer mixture produced by the die is cooled by blown air or water bath. The polymer mixture is then compression molded at 200°C. to a thickness dependent on a mold tool and its respective cavity. The resulting polymer mixture is shaped (*e.g.,* as a plaque or sheet) and exhibits homogenously distributed $\beta$TCP. The shaped polymer mixture is then subjected to an orientation or stretching process. In order to achieve the highest level of stretching/orientation, the shaped polymer mixture is stretched biaxially (along the x- and y-axes) to a stretch ratio. The oriented polymer mixture is then annealed at temperatures ranging from 80°C. to 200°C. This step allows the polymers comprising the polymer mixture to relax after the stretching process, thereby making the polymers amenable to the subsequent processing steps. The shaped and oriented polymer mixture is then cut into at least one strip, and the cut strip (or strips) is wrapped around a mandrel and placed in the cavity of a compression tool. The wrapped mandrel is compressed at a temperature ranging from about 5°C. to about 300°C., at a pressures of up to 282N/cm$^2$, and for a time of about 5 minutes to about 60 minutes so that the annular layers of the wound strip are laminated to each other. The laminated structure is then allowed to cool for about 5-30 minutes, whereafter a formed tissue integration device is removed from the compression tool. Thereafter, one or more pores or vented holes can be formed in the tissue integration device (*e.g.,* using a laser machining device). The vented holes or pores, along with the homogenous distribution of βTCP, promote tissue ingrowth into the tissue integration device once implanted in a subject.

**Example 2. Device comparison**

This Example illustrates a comparison between a tissue integration device prepared according to the present disclosure and a market leading predicate biocomposite device.

*Methods and materials*

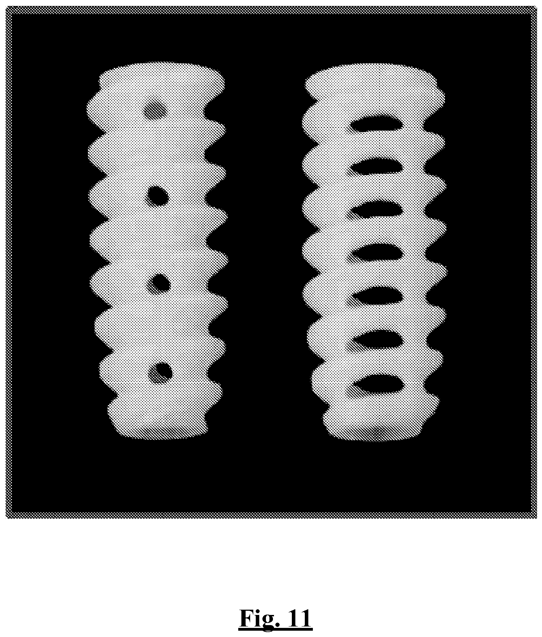

Comparative torsional strength, identified as a key measurement of clinical relevance, was measured between a tissue integration device prepared according to the present disclosure as a suture anchor, and a market leading predicate of equal material grade and geometry. The market leading suture anchor screw (85% PLLA/ 15% β-TCP; dimensions 5.5 mm Φ x 19.1 mm length) was used as a control. The control anchor screw (lot/reference #11858530) was produced by Arthrex, Inc. (Naples, FL). The tissue integration device was produced using a comparable material grade as the predicate, and to an identical gross geometry, with two different levels of porosity: one was equal to that of the predicate device (Tissue Integration Device 1); while the other contained a higher porosity to the predicate (Tissue Integration Device 2) (Fig. 11). Torsional strength was measured by inserting the screws into a synthetic solid bone block composed of high-density polyurethane foam, equivalent to cortical bone. Screws were inserted into a pre-drilled and tapped hole, using a drive mechanism which served to ensure consistent orthogonal alignment upon insertion, with inbuilt torque measurement capability. Failure torque and mode of failure were recorded for each screw.

*Results*

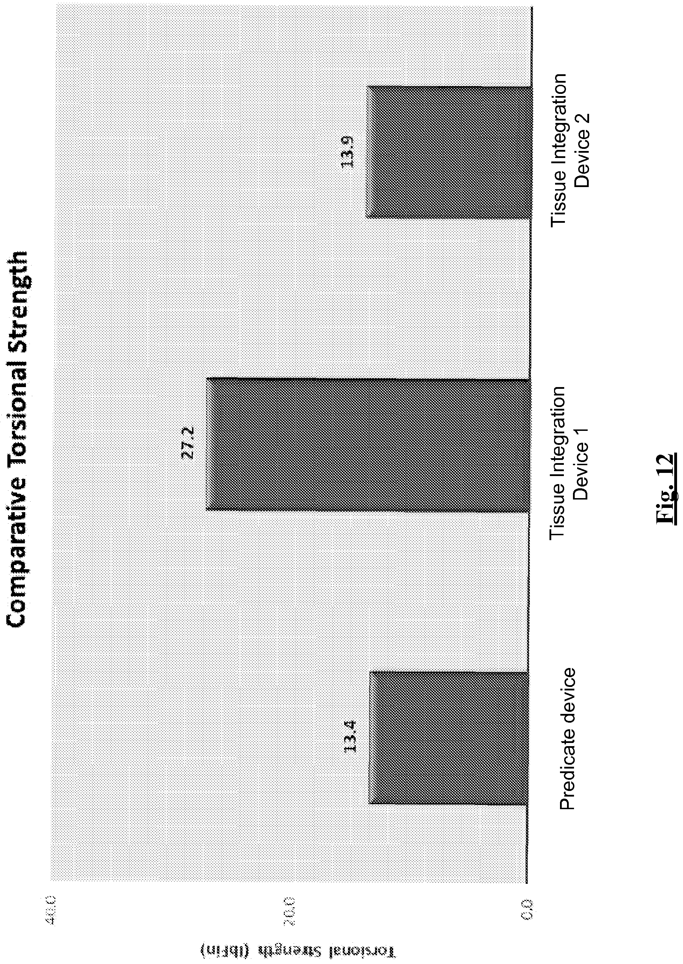

As shown in Table 1 and Fig. 12, torsional strength of Tissue Integration Device 1 showed an *increase* of 102% (27.2 lbF.in vs. 13.4 lbF. in, which corresponds to 3.07 Nm vs. 1.51 Nm), when compared to a predicate product of identical geometry and material grade (85% PLLA, 15% β-TCP). The tissue integration devices demonstrated a torsional shear failure mode, consistent with predicate devices. In addition, Tissue Integration Device 2 showed an increase of 3% (13.9 lbF.in vs. 13.4 lbF. in, which corresponds to 1.57 Nm vs. 1.51 Nm) when compared to the predicate produce while containing a significantly higher porosity as compared to the predicate. The porosity of Tissue Integration Device 2 was appropriately 100% greater when compared to the porosity of Tissue Integration Device 1 and the predicate product.

Table 1: Comparative torsonial strength of various biocomposite screws, of identical gross geometry and material grade. In the following table 1 lbF.in corresponds to 0.113 Nm.

| Specimen | Description | Torsional Strength (lbF.in) | | | | | |
|---|---|---|---|---|---|---|---|
| | | Average | Std Dev | n=1 | n=2 | n=3 | % increase |
| 1 | Predicate (Circular Pores: 7 x Ø1.02mm) | 13.4 | 0.80 | 13.5 | 14.2 | 12.6 | - |
| 2 | Tissue Integration Device 1 (Circular Pores: 7 x Ø1.02mm) | 27.2 | 0.98 | 26.6 | 28.3 | 26.6 | 102% |
| 3 | Tissue Integration Device 2 (Elliptical Pores: 14 x 0(1.02 x 2.03) mm, equivalent to PEEK porous design) | 13.9 | 1.40 | 12.5 | 15.3 | 13.9 | 3% |

From the above description of the present disclosure, those skilled in the art will perceive improvements, changes and modifications. Such improvements, changes, and modifications are within the skill of those in the art and are intended to be covered by the invention as long as they fall within the scope of the appended claims.

**Claims**

**1.**  A tissue integration device produced by the steps of:

forming a polymer containing mixture into a shape being one of a film, a strip, a sheet, and a plaque, the polymer containing mixture comprising a polymer resin and a growth-promoting medium;

orienting at least one polymer comprised in the polymer resin by stretching in at least one direction; forming the shaped polymeric material into the tissue integration device;

forming one or more line-of-sight pores in the tissue integration device, wherein the one or more line-of-sight pores are sized and dimensioned to promote tissue ingrowth when the tissue integration device is implanted in a subject, wherein two or more of the line-of- sight pores intersect with one another.

2. The tissue integration device of claim 1, wherein the at least one polymer is selected from the group consisting of a polylactide, a polyglycolide, an ultra-high-molecular- weight polyethylene, a polyether ether ketone, a polyurethane, a polytetrafluoroethylene, a polydioxanone, and combinations thereof.

3. The tissue integration device of claim 2, wherein the at least one polymer is a polylactide and the growth-promoting medium is β-tricalcium phosphate.

4. The tissue integration device of claim 1, wherein the step of forming a polymer containing mixture into a shape is done by processing the polymer containing mixture by at least one of extruding, casting, injection molding, transfer molding, thermoforming, rotational molding and blow molding, wherein the processed polymer containing mixture has a homogenous distribution of the growth-promoting medium.

5. The tissue integration device of claim 1, wherein the step of orienting at least one polymer comprising the polymer resin further includes stretching the shape under defined conditions until about the maximum stretch ratio of the at least one polymer is reached.

6. The tissue integration device of claim 1, wherein the shaped polymer containing mixture is stretched biaxially.

7. The tissue integration device of claim 6, wherein the shaped polymer containing mixture is stretched biaxially at a ratio that is not 1 :1.

8. The tissue integration device of claim 1, wherein the shaped polymer containing mixture is annealed to a specific target percentage after the orienting step.

9. The tissue integration device of claim 8, wherein the annealed, shaped polymer containing mixture is subjected to the following steps:

cutting the annealed, shaped polymer containing mixture into at least one strip;
wrapping the at least one strip around a mandrel to form a tube of concentric annular layers; and
compressing the layers together to laminate the layers.

10. The tissue integration device of claim 1, wherein the one or more pores are sized and dimensioned to promote tissue ingrowth when the tissue integration device is implanted in a subject.

11. The tissue integration device of claim 10, wherein the tissue ingrowth is vascular tissue ingrowth throughout all or a substantial portion of the tissue integration device.

12. The tissue integration device of any preceding claim, wherein the one or more line-of- sight pores are sized and dimensioned to contain one or more stem cells, one or more growth factors, and combinations thereof.

13. A method for forming a tissue integration device, the method comprising the steps of:

forming a polymer containing mixture that includes a polymer resin and a tissue growth- promoting medium;
processing the polymer containing mixture into a shape selected from the group consisting of a plaque, a sheet, a film and a strip;
orienting at least one polymer comprising the polymer resin by stretching in at least one direction;
annealing the shaped polymer containing mixture;
cutting the annealed, shaped polymer containing mixture into at least one strip;
wrapping the at least one strip around a mandrel to form a tube of concentric annular layers;
compressing the layers together to laminate the layers; and

forming two or more line of sight pores in the tissue integration device, wherein the two or more pores are sized and dimensioned to promote tissue ingrowth when the tissue integration device is implanted in a subject and wherein two or more of the line of line pores intersect with one another.

14. The method of claim 13, wherein the shaped polymer containing mixture is stretched biaxially.

15. The method of claim 13, wherein the step of forming a polymer containing mixture into a shape is done by processing the polymer containing mixture by at least one of extruding, casting, injection molding, transfer molding, thermoforming, rotational molding and blow molding, wherein the processed polymer containing mixture has a homogenous distribution of the tissue growth-promoting medium.

**Patentansprüche**

1. Gewebeintegrationsvorrichtung, die durch die folgenden Schritte hergestellt wird:

Formen eines polymerhaltigen Gemischs zu einer Form, die eines aus einer Folie, einem Streifen, einem Blatt und einer Platte ist, wobei das polymerhaltige Gemisch ein Polymerharz und ein wachstumsförderndes Mittel umfasst;
Ausrichten mindestens eines Polymers, das in dem Polymerharz umfasst ist, durch Strecken in mindestens eine Richtung;
Formen des geformten polymeren Materials zu der Gewebeintegrationsvorrichtung;
Formen einer oder mehrerer Sichtlinienporen in der Gewebeintegrationsvorrichtung, wobei die eine oder die mehreren Sichtlinienporen so bemessen und dimensioniert sind, dass sie das Gewebeeinwachsen fördern, wenn die Gewebeintegrationsvorrichtung in einem Individuum implantiert ist, wobei zwei oder mehr der Sichtlinienporen einander schneiden.

2. Gewebeintegrationsvorrichtung nach Anspruch 1, wobei das mindestens eine Polymer aus der Gruppe ausgewählt ist, die aus einem Polylactid, einem Polyglycolid, einem Polyethylen mit ultrahoher Molekülmasse, einem Polyetheretherketon, einem Polyurethan, einem Polytetrafluorethylen, einem Polydioxanon und Kombinationen davon besteht.

3. Gewebeintegrationsvorrichtung nach Anspruch 2, wobei das mindestens eine Polymer ein Polylactid ist und das wachstumsfördernde Mittel β-Tricalciumphosphat ist.

4. Gewebeintegrationsvorrichtung nach Anspruch 1, wobei der Schritt des Formens eines polymerhaltigen Gemischs in eine Form durch Verarbeiten des polymerhaltigen Gemischs mit mindestens einem aus Extrudieren, Gießen, Spritzgießen, Spritzpressen, Thermoformen, Rotationsgießen und Spritzblasen erfolgt, wobei das verarbeitete polymerhaltige Gemisch eine homogene Verteilung des wachstumsfördernden Mittels aufweist.

5. Gewebeintegrationsvorrichtung nach Anspruch 1, wobei der Schritt des Ausrichtens mindestens eines Polymers, das das Polymerharz umfasst, ferner das Strecken der Form unter definierten Bedingungen, bis etwa das maximale Streckverhältnis des mindestens einen Polymers erreicht wird, beinhaltet.

6. Gewebeintegrationsvorrichtung nach Anspruch 1, wobei das geformte polymerhaltige Gemisch biaxial gestreckt wird.

7. Gewebeintegrationsvorrichtung nach Anspruch 6, wobei das geformte polymerhaltige Gemisch biaxial in einem Verhältnis, das nicht 1:1 ist, gestreckt wird.

8. Gewebeintegrationsvorrichtung nach Anspruch 1, wobei das geformte polymerhaltige Gemisch nach dem Ausrichtungsschritt bis zu einem spezifischen Zielprozentsatz getempert wird.

9. Gewebeintegrationsvorrichtung nach Anspruch 8, wobei das getemperte, geformte polymerhaltige Gemisch den folgenden Schritten unterzogen wird:

Schneiden des getemperten, geformten polymerhaltigen Gemischs in mindestens einen Streifen;
Wickeln des mindestens einen Streifens um einen Dorn, um eine Röhre aus konzentrischen kreisförmigen Schichten zu bilden; und
Zusammenpressen der Schichten, um die Schichten zu laminieren.

**10.** Gewebeintegrationsvorrichtung nach Anspruch 1, wobei die eine oder die mehreren Poren so bemessen und dimensioniert sind, dass sie das Gewebeeinwachsen fördern, wenn die Gewebeintegrationsvorrichtung in ein Individuum implantiert ist.

**11.** Gewebeintegrationsvorrichtung nach Anspruch 10, wobei das Gewebeeinwachsen ein vaskuläres Gewebeeinwachsen über die gesamte oder einen wesentlichen Teil der Gewebeintegrationsvorrichtung ist.

**12.** Gewebeintegrationsvorrichtung nach einem der vorstehenden Ansprüche, wobei die eine oder die mehreren Sichtlinienporen so bemessen und dimensioniert sind, dass sie eine oder mehrere Stammzellen, einen oder mehrere Wachstumsfaktoren und Kombinationen davon enthalten.

**13.** Verfahren zum Herstellen einer Gewebeintegrationsvorrichtung, wobei das Verfahren die folgenden Schritte umfasst:

Formen eines polymerhaltigen Gemischs, das ein Polymerharz und ein gewebewachstumsförderndes Mittel beinhaltet;
Verarbeiten des polymerhaltigen Gemischs zu einer Form, die aus der Gruppe ausgewählt ist, die aus einer Platte, einem Blatt, einer Folie und einem Streifen besteht;
Ausrichten mindestens eines Polymers, das das Polymerharz umfasst, durch Strecken in mindestens eine Richtung;
Tempern des geformten polymerhaltigen Gemischs;
Schneiden des getemperten, geformten polymerhaltigen Gemischs in mindestens einen Streifen;
Wickeln des mindestens einen Streifens um einen Dorn, um eine Röhre aus konzentrischen kreisförmigen Schichten zu bilden;
Zusammenpressen der Schichten, um die Schichten zu laminieren; und
Formen von zwei oder mehreren Sichtlinienporen in der Gewebeintegrationsvorrichtung, wobei die zwei oder die mehreren Poren so bemessen und dimensioniert sind, dass sie das Gewebeeinwachsen fördern, wenn die Gewebeintegrationsvorrichtung in einem Individuum implantiert ist, und wobei zwei oder mehr der Sichtlinienporen einander schneiden.

**14.** Verfahren nach Anspruch 13, wobei das geformte polymerhaltige Gemisch biaxial gestreckt wird.

**15.** Verfahren nach Anspruch 13, wobei der Schritt des Formens eines polymerhaltigen Gemischs in eine Form durch Verarbeiten des polymerhaltigen Gemischs mit mindestens einem aus Extrudieren, Gießen, Spritzgießen, Spritzpressen, Thermoformen, Rotationsgießen und Spritzblasen erfolgt, wobei das verarbeitete polymerhaltige Gemisch eine homogene Verteilung des wachstumsfördernden Mittels aufweist.

**Revendications**

**1.** Dispositif d'intégration tissulaire produit par les étapes :

de formation d'un mélange contenant un polymère en une forme qui est l'une parmi un film, une bande, une feuille et une plaque, le mélange contenant un polymère comprenant une résine polymère et un milieu favorisant la croissance ;
d'orientation d'au moins un polymère compris dans la résine polymère par étirage dans au moins une direction ;
de formation du matériau polymère façonné en le dispositif d'intégration tissulaire ;
de formation d'un ou de plusieurs pores de ligne visuelle dans le dispositif d'intégration tissulaire, dans lequel les un ou plusieurs pores de ligne visuelle sont dimensionnés pour favoriser l'interposition tissulaire lorsque le dispositif d'intégration tissulaire est implanté chez un sujet, dans lequel deux des pores de ligne visuelle ou plus se croisent.

**2.** Dispositif d'intégration tissulaire selon la revendication 1, dans lequel l'au moins un polymère est choisi dans le groupe composé d'un polylactide, d'un polyglycolide, d'un polyéthylène à masse moléculaire ultra élevée, d'un polyéther éther cétone, d'un polyuréthane, d'un polytétrafluoroéthylène, d'une polydioxanone et leurs combinaisons.

**EP 3 203 930 B1**

3. Dispositif d'intégration tissulaire selon la revendication 2, dans lequel l'au moins un polymère est un polylactide et le milieu favorisant la croissance est du phosphate de β-tricalcium.

4. Dispositif d'intégration tissulaire selon la revendication 1, dans lequel l'étape de formation d'un mélange contenant un polymère en une forme est effectuée en traitant le mélange contenant un polymère par au moins l'un parmi une extrusion, une coulée, un moulage par injection, un moulage par transfert, un thermoformage, un rotomoulage et un moulage par soufflage, dans lequel le mélange contenant un polymère traité a une distribution homogène du milieu favorisant la croissance.

5. Dispositif d'intégration tissulaire selon la revendication 1, dans lequel l'étape d'orientation d'au moins un polymère comprenant la résine polymère comprend en outre l'étirage de la forme dans des conditions définies jusqu'à ce que le rapport d'étirage maximal de l'au moins un polymère soit atteint.

6. Dispositif d'intégration tissulaire selon la revendication 1, dans lequel le mélange contenant un polymère façonné est étiré biaxialement.

7. Dispositif d'intégration tissulaire selon la revendication 6, dans lequel le mélange contenant un polymère façonné est étiré biaxialement selon un rapport qui n'est pas 1:1.

8. Dispositif d'intégration tissulaire selon la revendication 1, dans lequel le mélange contenant un polymère façonné est recuit jusqu'à un pourcentage cible spécifique après l'étape d'orientation.

9. Dispositif d'intégration tissulaire selon la revendication 8, dans lequel le mélange contenant un polymère façonné recuit est soumis aux étapes suivantes :

découpe du mélange contenant un polymère façonné recuit en au moins une bande ;
enroulement de l'au moins une bande autour d'un mandrin pour former un tube de couches annulaires concentriques ; et compression des couches ensemble pour stratifier les couches.

10. Dispositif d'intégration tissulaire selon la revendication 1, dans lequel les un ou plusieurs pores ont une taille et des dimensions permettant de favoriser l'interposition tissulaire lorsque le dispositif d'intégration tissulaire est implanté chez un sujet.

11. Dispositif d'intégration tissulaire selon la revendication 10, dans lequel l'interposition tissulaire est une interposition tissulaire vasculaire dans la totalité ou une partie substantielle du dispositif d'intégration tissulaire.

12. Dispositif d'intégration tissulaire selon une quelconque revendication précédente, dans lequel les un ou plusieurs pores de ligne visuelle ont une taille et des dimensions permettant de contenir une ou plusieurs cellules souches, un ou plusieurs facteurs de croissance et leurs combinaisons.

13. Méthode de formation d'un dispositif d'intégration tissulaire, la méthode comprenant les étapes :

de formation d'un mélange contenant un polymère qui comprend une résine polymère et un milieu favorisant la croissance tissulaire ;
de traitement du mélange contenant un polymère en une forme choisie dans le groupe composé d'une plaque, d'une feuille, d'un film et d'une bande ;
d'orientation d'au moins un polymère comprenant la résine polymère par étirage dans au moins une direction ;
de recuisson du mélange contenant un polymère façonné ;
de découpe du mélange contenant un polymère façonné recuit en au moins une bande ;
d'enroulement de l'au moins une bande autour d'un mandrin pour former un tube de couches annulaires concentriques ;
de compression des couches ensemble pour stratifier les couches ; et
de formation de deux pores de ligne visuelle ou plus dans le dispositif d'intégration tissulaire, dans lequel les deux pores de ligne visuelle ou plus ont une taille et des dimensions permettant de favoriser l'interposition tissulaire lorsque le dispositif d'intégration tissulaire est implanté chez un sujet, et dans lequel deux ou des pores de ligne visuelle ou plus se croisent.

14. Méthode selon la revendication 13, dans lequel le mélange contenant un polymère façonné est étiré biaxialement.

**15.** Méthode selon la revendication 13, dans lequel l'étape de formation d'un mélange contenant un polymère en une forme est effectuée en traitant le mélange contenant un polymère par au moins l'un parmi une extrusion, une coulée, un moulage par injection, un moulage par transfert, un thermoformage, un rotomoulage et un moulage par soufflage, dans laquelle le mélange contenant un polymère traité a une distribution homogène du milieu favorisant la croissance tissulaire.

**10**

| FORM A POLYMER MIXTURE | — 12 |

↓

| SHAPE THE POLYMER MIXTURE | — 14 |

↓

| ORIENT THE SHAPED POLYMER MIXTURE | — 16 |

↓

| ANNEAL THE ORIENTED, SHAPED POLYMER MIXTURE | — 18 |

↓

| CUT THE ORIENTED, SHAPED POLYMER MIXTURE IN STRIP(S) | — 20 |

↓

| LAMINATE THE STRIP(S) | — 22 |

↓

| FORM PORE(S) | — 24 |

**Fig. 1**

**Fig. 2**

**Fig. 3**

**Fig. 4**

A

26

28

B

26

28

**Fig. 5**

**Fig. 6**

32    38

**Fig. 7**

**Fig. 8**

**Fig. 9**

A

30

10B

40

10B

B

30

42

40

44

α1  40'  44

α2  40''

**Fig. 10**

**Fig. 11**

# Comparative Torsional Strength

**Fig. 12**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2006141012 A1
- US 20110020292 A1, Van't Hof
- WO 2015052346 A1, Durkin